# EUROPÄISCHE PATENTSCHRIFT

(11) **EP 2 111 398 B1**
(45) Veröffentlichungstag und Bekanntmachung des Hinweises auf die Patenterteilung: **15.10.2014**
(21) Anmeldenummer: 08701022.9
(22) Anmeldetag: 09.01.2008
(51) Int. Cl.: C07D 307/94, C07D 407/12

(54) **SPIROCYCLISCHE TETRONSÄURE-DERIVATIVE**
SPIROCYCLIC TETRONIC ACID DERIVATIVES
DÉRIVÉS SPIROCYCLIQUES DE L'ACIDE TÉTRONIQUE

(30) Priorität: 12.01.2007 DE 102007001866
(43) Veröffentlichungstag der Anmeldung: 28.10.2009
(73) Patentinhaber: Bayer CropScience AG, 40789 Monheim (DE)
(72) Erfinder: FISCHER, Reiner, 40789 Monheim (DE); BRETSCHNEIDER, Thomas, 53797 Lohmar (DE); SCHULTE, Hubertus, 41812 Erkelenz (DE); KRAUTSTRUNK, Gerhard, 40789 Monheim (DE); ARNOLD, Christian, 40764 Langenfeld (DE); SANWALD, Erich, 24159 Kiel (DE)
(74) Vertreter: BIP Patents
(86) Internationale Anmeldenummer: PCT/EP2008/000084
(87) Internationale Veröffentlichungsnummer: WO 2008/083950

(56) Entgegenhaltungen:
- EP-A- 0 528 156
- WO-A-00/42850

## Beschreibung

Die vorliegende Erfindung betrifft neue spirocyclische Tetronsäure-Derivate, mehrere Verfahren zu ihrer Herstellung und ihre Verwendung als Schädlingsbekämpfungsmittel.

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 00/42850, WO 01/17972, WO 01/23354, WO 01/74770, WO 03/013 249, WO 04/024 688, WO 04/080 962, WO 04/111 042, WO 05/092897, WO 06/000355, WO 06/029799, WO 06/089633, WO 07/048545 und WO 07/073856 bekannt.

Die akarizide und/oder insektizide Wirksamkeit und/oder akarizide und/oder insektizide Wirkungsbreite dieser Verbindungen ist jedoch insbesondere bei niedrigen Aufwandmengen und Konzentrationen nicht immer völlig zufriedenstellend. Weiterhin ist die Pflanzenverträglichkeit dieser Verbindungen gegenüber den Kulturpflanzen nicht immer ausreichend. Außerdem sind die toxikologischen Eigenschaften und/oder Umwelteigenschaften dieser Verbindungen nicht immer völlig zufriedenstellend.

Es wurde nun eine neue Verbindung der Formel (I-3)

Man erhält die Verbindung der Formel (I-3), wenn man die Verbindung (II) 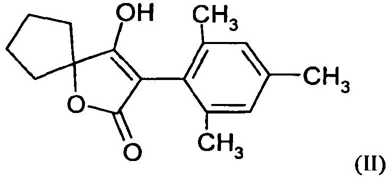 jeweils
A α) mit Verbindungen der Formel (III) 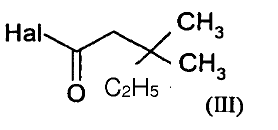 in welcher
- Hal: für Halogen (insbesondere Chlor oder Brom) steht
oder
A β) mit Carbonsäureanhydriden der Formel (IV) 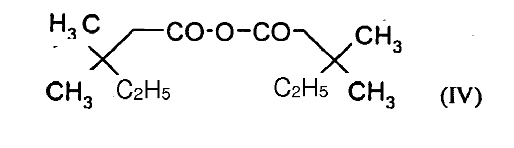 gegebenenfalls in Gegenwart eines Verdünnungsmittel und gegebenenfalls in Gegenwart eines Säurebindemittels umsetzt;
oder
A γ) mit aktivierten Carbonsäurederivaten der Formel (V) 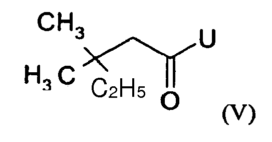 in welcher
- U: für eine durch Carbonsäureaktivierungsreagenzien wie Carbonyldiimidazol, Carbonyldiimide (wie z. B. Dicyclohexylcarbondiimid), Phosphorylierungs-reagenzien (wie z. B. POCI3, BOP-Cl), Halogenierungsmittel, wie z. B. Thionylchlorid, Oxalychlorid, Phosgen oder Chlorameisensäureester eingeführte Abgangsgruppe steht,
gegebenenfalls in Gegenwart eines Verdünnungsmittels und gegebenenfalls in Gegenwart eines Säurebindemittels umsetzt.

Die beim erfindungsgemäßen Verfahren (A) als Ausgangsstoffe benötigte Verbindung (II) ist bekannt aus EP-A-528 156, Beispiel I-a-33.

Die beim erfindungsgemäßen Verfahren (A) als Ausgangsstoffe benötigte Carbonsäurechloride (III), Carbonsäureanhydride (IV) und Carbonsäurederivate (V) sind teilweise käuflich oder lassen sich nach allgemein bekannten Methoden (s. Herstellungsbeispiele) herstellen.

Das Verfahren (A-α) ist dadurch gekennzeichnet, dass man die Verbindung der Formel (II) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (A-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (A-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (A-α) innerhalb eines größeren Bereiches variiert werden. Im allgemeinen arbeitet man bei Temperaturen zwischen - 20°C und +150°C, vorzugsweise zwischen 0°C und 100°C.

Bei der Durchführung des erfindungsgemäßen Verfahrens (A-α) wird der Ausgangsstoff der Formel (II) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (A-β) ist dadurch gekennzeichnet, dass man die Verbindung der Formel (II) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (A-β) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (A-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (A-β) innerhalb eines größeren Bereiches variiert werden. Im allgemeinen arbeitet man bei Temperaturen zwischen - 20°C und +150°C, vorzugsweise zwischen 0°C und 100°C.

Bei der Durchführung des erfindungsgemäßen Verfahrens (A-β) wird der Ausgangsstoff der Formel (II) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (A-γ) ist dadurch gekennzeichnet, daß man die Verbindung der Formel (II) jeweils mit aktivierten Carbonsäurederivaten der Formel (V) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (A-γ) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (A-γ) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (A-y) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (A-γ) wird im allgemeinen unter Normaldruck durchgeführt.

Bei der Durchführung des erfindungsgemäßen Verfahrens (A-γ) wird der Ausgangsstoff der Formel (II) und das entsprechende aktivierte Carbonsäurederivat der Formel (V) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracuneulus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyraustanubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Objane der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren der Nucleinsäure Synthese
Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
Benomyl, Carbendazim, Diethofencarb, Fuberidazole, Pencycuron, Thiabendazol, Thiophanatmethyl, Zoxamid
Inhibitoren der Atmungskette Komplex I
Diflumetorim
Inhibitoren der Atmungskette Komplex II
Boscalid, Carboxin, Fenfuram, Flutolanil, Furametpyr, Mepronil, Oxycarboxin, Penthiopyrad, Thifluzamid
Inhibitoren der Atmungskette Komplex III
Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
Dinocap, Fluazinam
Inhibitoren der ATP Produktion
Fentinacetat, Fentinchlorid, Fentinhydroxid, Silthiofam
Inhibitoren der Aminosäure- und Proteinbiosynthese
Andoprim, Blasticidin-S, Cyprodinil, Kasugamycin, Kasugamycinhydrochlorid Hydrat, Mepanipyrim, Pyrimethanil
Inhibitoren der Signal-Transduktion
Fenpiclonil, Fludioxonil, Quinoxyfen
Inhibitoren der Fett- und Membran Synthese
Chlozolinat, Iprodion, Procymidon, Vinclozolin
Ampropylfos, Kalium-Ampropylfos, Edifenphos, Iprobenfos (IBP), Isoprothiolan, Pyrazophos
Tolclofos-methyl, Biphenyl
Iodocarb, Propamocarb, Propamocarb hydrochlorid
Inhibitoren der Ergosterol Biosynthese
Fenhexamid,
Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsülfat, Oxpoconazol, Fenarimol, Flurprimidol, Nuarimol, Pyrifenox, Triforin, Pefurazoat, Prochloraz, Triflumizol, Viniconazol,
Aldimorph, Dodemorph, Dodemorphacetat, Fenpropimorph, Tridemorph, Fenpropidin, Spiroxamin,
Naftifin, Pyributicarb, Terbinafin
Inhibitoren der Zellwand Synthese
Benthiavalicarb, Bialaphos, Dimethomorph, Flumorph, Iprovalicarb, Polyoxins, Polyoxorim, Validamycin A
Inhibitoren der Melanin Biosynthese
Capropamid, Diclocymet, Fenoxanil, Phtalid, Pyroquilon, Tricyclazol
Resistenzinduktion
Acibenzolar-S-methyl, Probenazol, Tiadinil
Multisite
Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[l,5-a]pyrimidin-7-amine, N-[1-(5-Brom-3-chloropyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropylmethoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R-isomer), Tralomethrin, Transfluthrin, ZXI 8901, Pyrethrins (pyrethrum)
DDT
   Oxadiazine,
      zum Beispiel Indoxacarb
   Semicarbazon,
      zum Beispiel Metaflumizon (BAS3201)
Acetylcholin-Rezeptor-Agonisten/-Antagonisten
   Chloronicotinyle,
   zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
Spinosyne,
   zum Beispiel Spinosad
GABA-gesteuerte Chlorid-Kanal-Antagonisten
   Organochlorine,
   zum Beispiel Camphechlor, Chlordane, Endosulfan, Gamma-HCH, HCH, Heptachlor, Lindane, Methoxychlor
   Fiprole,
   zum Beispiel Acetoprole, Ethiprole, Fipronil, Pyrafluprole, Pyriprole, Vaniliprole
Chlorid-Kanal-Aktivatoren
Mectine,
   zum Beispiel Abamectin, Emamectin, Emamectin-benzoate, Ivermectin, Lepimectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
Diacylhydrazine,
   zum Beispiel Chromafenozide, Halofenozide, Methoxyfenozide, Tebufenozide
Inhibitoren der Chitinbiosynthese
   Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
   Pyrrole,
   zum Beispiel Chlorfenapyr
   Dinitrophenole,
   zum Beispiel Binapacyrl, Dinobuton, Dinocap, DNOC, Meptyldinocap
Seite-I-Elektronentransportinhibitoren
   METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
Rotenone
Seite-III-Elektronentransportinhibitoren
Acequinocyl, Fluacrypyrim
Mikrobielle Disruptoren der Insektendarmmembran
Bacillus thuringiensis-Stämme
Inhibitoren der Fettsynthese
Tetronsäuren,
zum Beispiel Spirodiclofen, Spiromesifen,
Tetramsäuren,
zum Beispiel Spirotetramat, cis-3-(2,5-dimethylphenyl)-4-hydroxy-8-methoxy-1-azaspiro[4.5]dec-3-en-2-on
Carboxamide,
zum Beispiel Flonicamid
Oktopaminerge Agonisten,
zum Beispiel Amitraz
Inhibitoren der Magnesium-stimulierten ATPase,
Propargite
Nereistoxin-Analoge,
zum Beispiel Thiocyclam hydrogen oxalate, Thiosultap-sodium
Agonisten des Ryanodin-Rezeptors,
Benzoesäuredicarboxamide,
zum Beispiel Flubendiamid
Anthranilamide,
zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
Biologika, Hormone oder Pheromone
Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
   zum Beispiel Aluminium phosphide, Methyl bromide, Sulfuryl fluoride
   Fraßhemmer,
   zum Beispiel Cryolite, Flonicamid, Pymetrozine
   Milbenwachstumsinhibitoren,
   zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenem, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirexjuvencus, Urocerus gigas, Urocerus gigas taignus, Urocerus augur,
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosujilasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Wirkstoffe geht aus den nachfolgenden Beispielen hervor.

### Herstellungsbeispiele

### Beispiel (I-1) Verfahren A (α)

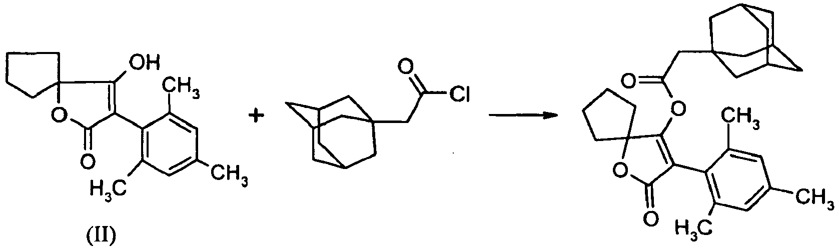

0,545g (2 mmol) der Verbindung II werden in 50 ml Dichlormethan vorgelegt, bei Raumtemperatur 0,223g (2,2 mmol) Triethylamin zugegeben, bei 0-10°C 0,447g (2,1 mmol) Adamantylessigsäurechlorid zugetropft und über Nacht bei Raumtemperatur verrührt. Zur Aufarbeitung wird der Ansatz mit verdünnter Citronensäure und 5%iger NaOH ausgeschüttelt, die organische Phase getrocknet, eingeengt und mittels Säulenchromatographie (Kieselgel, Dichlormethan) gereinigt.
Ausbeute: 0,656g (73% d.Th.), logP = 6,94

### Beispiel (1-2) Verfahren A (γ)

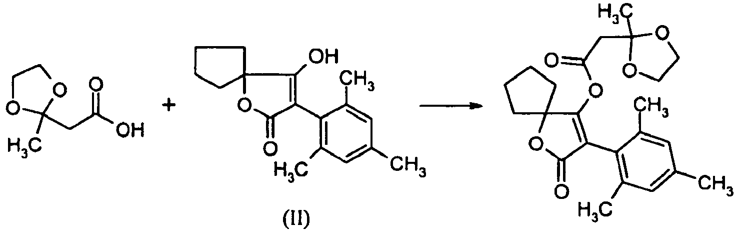

0,5g (3,42 mmol) Acetessigsäure-diethylenglykol-ketal werden in 30 ml Acetonitril vorgelegt, 1,327g (10,26 mmol) N,N-Diisopropylethylamin zugesetzt, bei Raumtemperatur 1,045g (4,11 mmol) Bis(2-oxo-3-oxazolidinyl)phosphonsäurechlorid (BOP-C1) zugesetzt, 20 min nachgerührt, 1,118g (4,11 mmol) der Verbindung II zugetropft, über Nacht bei Raumtemperatur verrührt, der Ansatz eingeengt und mittels Säulenchromatographie (Kieselgel, Cyclohexan/Essigester) gereinigt.
Ausbeute: 0,117g (9% d.Th.), logP = 3,85

In Analogie zu Beispiel (I-1) erhält man **Beispiel (I-3)**

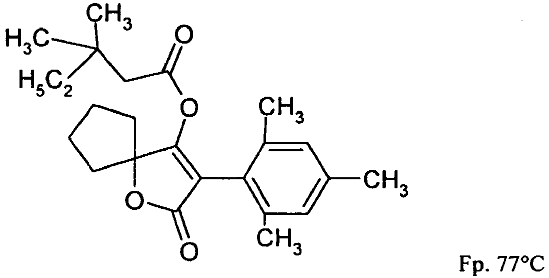

In Analogie zu Beispiel (1-2) erhält man **Beispiel (I-4)**

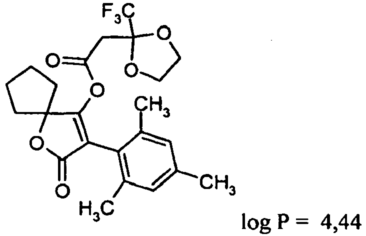

### Beispiel (I-5)

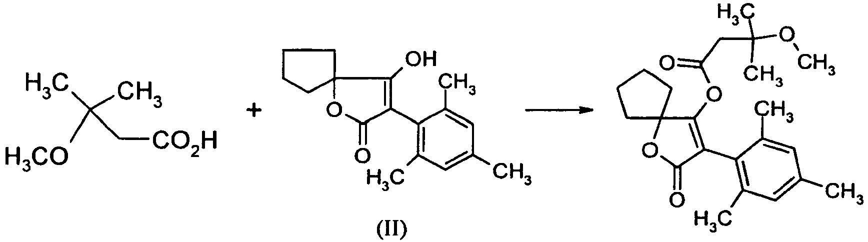

66 mg (0,5 mmol) 3-Methoxy-3-methyl-buttersäure, 0,17 ml (1,2 mmol) Triethylamin, 136 mg (0,5 mmol) der Verbindung II und 153 mg (0,6 mmol) 2-Chlor-1-methylpyridiniumchlorid werden über Nacht bei Raumtemperatur in 5 ml Tetrahydrofuran verrührt, der Ansatz eingeengt und mittels Säulenchromatographie (Kieselgel, Cyclohexan/Essigester) gereinigt.
Ausbeute: 149 mg (70% d.Th.), logP = 4,45

### Bestimmung der LogP-Werte

Die Bestimmung der in der Tabelle angegebenen logP-Werte erfolgte gemäß EEC-Directive 79/831 Annex V.A8 durch HPLC (High Performance Liquid Chromatography) an einer Phasenumkehrsäule (C18). Temperatur: 43°C

Eluenten für die Bestimmung im sauren Bereich (pH 2,3): 0,1 % wässrige Phosphorsäure, Acetonitril; linearer Gradient von 10% Acetonitril bis 90 % Acetonitril

Die Eichung erfolgte mit unverzweigten Alkan-2-onen (mit 3 bis 16 Kohlenstoffatomen), deren logP-Werte bekannt sind (Bestimmung der logP-Werte anhand der Rententionszeiten durch lineare Interpolation zwischen zwei aufeinander folgenden Alkanonen).

Die lambda-max-Werte wurden anhand der UV-Spektren von 200 nm bis 400 nm in den Maxima der chromatographischen Signale ermittelt.

### Beispiel 1

### Tetranychus urticae (TETRUR RAUS)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Buschbohnenpflanzen (*Phaseolus vulgaris*), die mit einer gemischten Population mit der Gemeinen resistenten Spinnmilbe (*Tetranychus urticae, resistent)* befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigt z. B. die folgende Verbindung der Herstellungsbeispiele überlegene Wirksamkeit bei einer Konzentration von 5ppm gegenüber dem Stand der Technik Beispiel I aus WO 00/42850 (Spiromesifen): I-3 (97%) gegenüber 30% SdT

### Beispiel 2

### Phaedon-Test (PHAECO Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit mit einer Aufwandmenge 100g/ha gegenüber dem Stand der Technik Beispiel I aus WO 00/42850 (Spiromesifen): I-2 (67%) gegenüber 33% SdT

### Beispiel 3

### Frankliniella occidentalis (FRANOC Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen werden diese in einer Konzentration von 1000 ppm nach dem Verdünnen jeweils der fertigen Präparatelösung zupipettiert.

Bohnenblattscheiben *(Phaseolus vularis),* die von einer gemischten Thripspopulation *(Frankliniella occidentalis)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Thripse abgetötet wurden; 0 % bedeutet, dass keine Thripse abgetötet wurden.

Bei diesem Test zeigen z.B. die folgenden Verbindungen der Herstellungsbeispiele überlegene Wirksamkeit mit einer Aufwandmenge 100 g/ha gegenüber dem Stand der Technik Beispiel I aus WO 00/42850 (Spiromesifen): I-2 (63 %) gegenüber 0 % SdT;
mit einer Aufwandmenge 500 g/ha gegenüber dem Stand der Technik Beipiel I aus WO 00/42850 (Spiromesifen): I-1 (80 %) gegenüber 0 % SdT.

### Beispiel 4

### Myzus persicae -Test (MYZUPE)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert.

Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeipiele überlegende Wirksamkeit gegenüber dem Stand der Technik: siehe Tabelle

| | | **MYZUPE** | |
|---|---|---|---|
| **Wirkstoff** | | ppm | % 6 d |
| **Beispiel I-5** | erfindungsgemäß | 100 | 55 |
| **Beispiel I aus** WO 00/42850 **(Spiromesifen)** | Stand der Technik | 100 | 30 |

### Beispiel 5

### Tetranychus-Test, OP-resistent/Tauchbehandlung (TETRUR)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert.

Bohnenpflanzen *(Phaseolus vulgaris),* die stark von allen Stadien der Gemeinen Spinnmilbe *(Tetranychus urticae sensibel)* befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindungen der Herstellungsbeispiele überlegene Wirksamkeit gegenüber dem Stand der Technik: siehe Tabelle

| | | **TETRUR** | |
|---|---|---|---|
| **Wirkstoff** | | ppm | % 7 d |
| **Beispiel 1-5** | erfindungsgemäß | 3,125 | 90 |
| | | 1,5625 | 50 |
| | | 0,78125 | 30 |
| **Beispiel I aus** WO 00/42850 **(Spiromesifen)** | Stand der Technik | 3,125 | 50 |
| | | 1,5625 | 30 |
| | | 0,78125 | 0 |

## Patentansprüche

1. Verbindung der Formel (I-3)

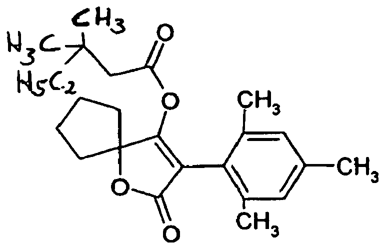

2. Verfahren zur Herstellung von Verbindung der Formel I-3 gemäß Anspruch 1 **dadurch gekennzeichnet, dass** man die Verbindung (II) 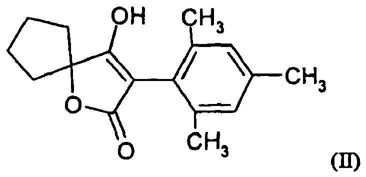 jeweils
α) mit Verbindungen der Formel (III) 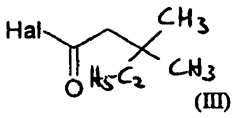 in welcher
Hal für Halogen steht
oder
β) mit Carbonsäureanhydriden der Formel (IV) 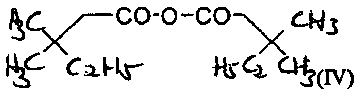 in welcher
Gegebenenfalls in Gegenwart eines Verdünnungsmittel und gegebenenfalls in Gegenwart eines Säurebindemittels umsetzt;
oder
γ) mit aktivierten Carbonsäurederivaten der Formel (V) 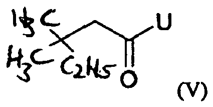 in welcher
U für eine durch Carbonsäureaktivierungsreagenzien wie Carbonyldiimidazol, Carbonyldiimide, Phosphorylierungs-reagenzien, Halogenierungsmittel, Phosgen oder Chlorameisensäureester eingeführte Abgangsgruppe steht,
gegebenenfalls in Gegenwart eines Verdünnungsmittels und gegebenenfalls in Gegenwart eines Säurebindemittels umsetzt.

3. Verwendung von Verbindung der Formel I-3 gemäß Anspruch 1 zur Herstellung von Schädlingsbekämpfungsmitteln gegen Tetranychus urticae.

4. Schädlingsbekämpfungsmittel gegen Tetranychus urticae, **gekennzeichnet durch** einen Gehalt an einer Verbindung der Formel I-3 gemäß Anspruch 1.

5. Verfahren zur Bekämpfung von tierischen Schädlingen, **dadurch gekennzeichnet, dass** man Verbindunge der Formel I-3 gemäß Anspruch 1 auf Tetranychus urticae und/oder ihren Lebensraum einwirken lässt.

6. Verwendung von Verbindung der Formel I-3 gemäß Anspruch 1 zur Bekämpfung von Tetranychus urticae.

7. Verfahren zur Herstellung von Schädlingsbekämpfungsmitteln gegen Tetranychus urticae, **dadurch gekennzeichnet, dass** man Verbindung der Formel I-3 gemäß Anspruch 1 mit Streckmitteln und/oder oberflächenaktiven Stoffen vermischt.

## Claims

1. Compound of the formula (I-3)

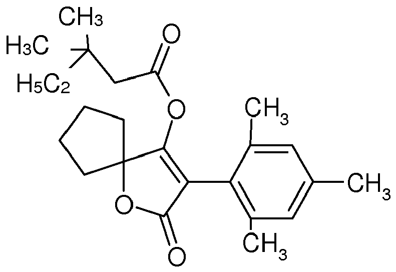

2. Process for preparing the compound of the formula (I-3) according to Claim 1, **characterized in that** the compound (II) 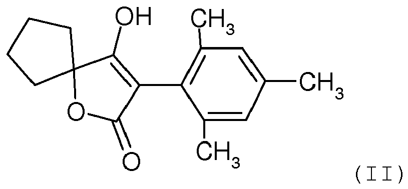 is in each case reacted
α) with compounds of the formula (III) 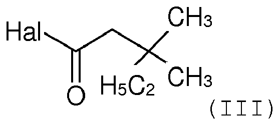 in which
Hal represents halogen
or
β) with carboxylic anhydrides of the formula (IV) 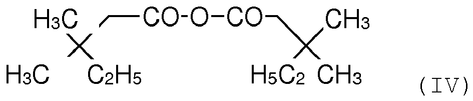 if appropriate in the presence of a diluent and if appropriate in the presence of an acid binder;
or
γ) with activated carboxylic acid derivatives of the formula (V) 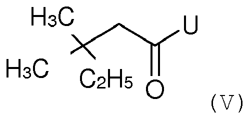 in which
U represents a leaving group introduced by carboxylic acid-activating reagents such as carbonyldiimidazole, carbonyldiimides, phosphorylating agents, halogenating agents, phosgene or chloroformic esters,
if appropriate in the presence of a diluent and if appropriate in the presence of an acid binder.

3. Use of the compound of the formula (I-3) according to Claim 1 for preparing pesticides against Tetranychus urticae.

4. Pesticides against Tetranychus urticae, **characterized in that** they comprise a compound of the formula (I-3) according to Claim 1.

5. Method for controlling animal pests, **characterized in that** compounds of the formula (I-3) according to Claim 1 are allowed to act on Tetranychus urticae and/or its habitat.

6. Use of compounds of the formula (I-3) according to Claim 1 for controlling Tetranychus urticae.

7. Process for preparing pesticides against Tetranychus urticae, **characterized in that** compounds of the formula (I-3) according to Claim 1 are mixed with extenders and/or surfactants.

## Revendications

1. Composé de formule (I-3)

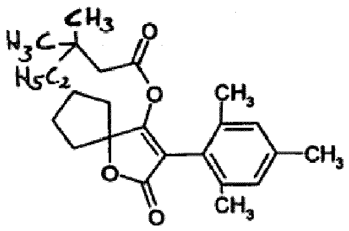

2. Procédé pour la préparation du composé de formule I-3 selon la revendication 1, **caractérisé en ce qu'**on fait réagir le composé (II) 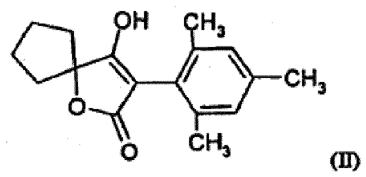 respectivement
α) avec des composés de formule (III) 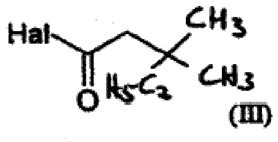 dans laquelle
Hal représente un atome d'halogène
ou
β) avec des anhydrides d'acides carboxyliques de formule (IV) 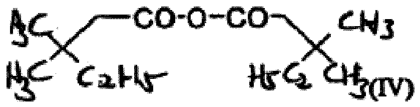 éventuellement en présence d'un diluant et éventuellement en présence d'un capteur d'acide ;
ou
γ) avec des dérivés d'acides carboxyliques activés de formule (V) 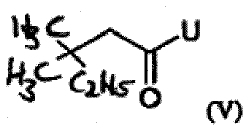 dans laquelle
U représente un groupe partant introduit au moyen de réactifs d'activation d'acides carboxyliques, tels que le carbonyldiimidazole, des carbonyldiimides, des réactifs de phosphorylation, des agents d'halogénation, le phosgène ou des esters d'acide chloroformique,
éventuellement en présence d'un diluant et éventuellement en présence d'un capteur d'acide.

3. Utilisation du composé de formule I-3 selon la revendication 1, pour la fabrication de pesticides contre *Tetranychus urticae.*

4. Pesticide contre *Tetranychus urticae,* **caractérisé par** une teneur en un composé de formule I-3 selon la revendication 1.

5. Procédé pour la lutte contre des ravageurs animaux, **caractérisé en ce qu'**on fait agir sur des *Tetranychus urticae* et/ou leur habitat le composé de formule I-3 selon la revendication 1.

6. Utilisation du composé de formule I-3 selon la revendication 1, pour la lutte contre *Tetranychus urticae.*

7. Procédé pour la fabrication de pesticides contre *Tetranychus urticae,* **caractérisé en ce qu'**on mélange le composé de formule I-3 selon la revendication 1 avec des excipients et/ou des substances tensioactives.
